# FASCICULE DE BREVET EUROPEEN

(11) **EP 0 477 113 B1**
(45) Date de publication et mention de la délivrance du brevet: **03.01.1996**
(21) Numéro de dépôt: 91420329.4
(22) Date de dépôt: 17.09.1991
(51) Int. Cl.: A61F 2/36

(54) **Ensemble prothétique de la hanche**
Prothesenteile für Hüftgelenk
Prosthetic assembly for hip joint

(30) Priorité: 18.09.1990 FR 9011766
(43) Date de publication de la demande: 25.03.1992
(73) Titulaire: MEDINOV SA, F-42312 Roanne Cedex (FR)
(72) Inventeur: Arnould, Hervé, F-01000 Bourg en Bresse (FR); Chambaud, Denis, F-42153 Riorges (FR); Dufaud, Jean-Louis, F-83300 Draguignan (FR); Grobost, Jérome, F-72530 Yvre l'Evêque (FR); Lafay, Jean-Pierre, F-90340 Chevremont (FR); Lucet, Alain, F-21850 Apollinaire (FR); Moati, Jean-Claude, F-92130 Issy les Moulineaux (FR); Petitjean, Dominique, F-01300 Belley (FR); Frick, Michel, Résidence Michelet Saint-Jacques, F-13009 Marseille (FR); Colombier, Michel, F-42153 Riorges (FR)
(74) Mandataire: Dupuis, François

(56) Documents cités:
- EP-A- 0 025 835
- DE-A- 3 247 726
- FR-A- 2 629 707
- US-A- 2 934 065

## Description

L'invention concerne le domaine des implants orthopédiques et plus particulièrement des prothèses de hanches.

Quel que soit le mode d'impaction des prothèses, notamment des tiges fémorales, avec ou sans ciment, le but recherché est d'obtenir une fixation la plus sûre, la plus efficace et la plus stable dans le temps. Il est par ailleurs apparu que c'est au niveau de la métaphyse de l'os que la fixation doit être particulièrement bien adaptée.

De nombreuses solutions ont été proposées pour tenter d'obtenir une fixation métaphysaire sûre et efficace. Les tiges fémorales sont exécutées selon différentes tailles pour tenter d'optimiser le remplissage métaphysaire, ces tailles varient selon une progression homothétique. Il n'est cependant pas tenu compte de la forte variabilité morphologique du fémur d'un individu sur l'autre.

Une étude radiologique et tomodensitométrique du fémur, en intégrant les règles de biomécanique, de stabilité et de transmission des efforts, fait apparaitre qu'il existe en réalité deux grandes familles de fémurs, à savoir les fémurs sensiblement rectilignes, ou droits, et les fémurs courbes, c'est-à-dire à large courbure interne. Bien évidemment, pour chacune de ces familles, le fémur présente différentes tailles.

Comme déjà indiqué, les nombreux modèles de tiges fémorales actuellement sur le marché offrent une gamme d'implants dont les tailles progressent d'une manière homothétique, mais ne tiennent pas compte de cette différence essentielle au niveau de la morphologie même du fémur. Cet état de la technique peut être illustré par l'enseignement du brevet FR-A-2629707 qui divulgue un équipement pour la réalisation d'une prothèse de hanche comprenant une série de pièces fémorales, différentes les unes des autres par la longueur de la tige et/ou les dimensions de ses sections transversales et/ou la longueur du col.

Le remplissage métaphysaire ne peut donc pas être considéré comme parfait, notamment dans le cas de fémurs à large courbure interne. Il en est de même, par conséquent, en ce qui concerne la stabilité de l'implant.

Selon l'invention, pour remédier à ces inconvénients, il a été conçu et mis au point un ensemble prothétique à partir d'une gamme de tiges fémorales dont les formes et dimensions ont été déterminées pour résoudre les problèmes suivants :
- optimisation de remplissage métaphysaire pour augmenter la stabilité de la tige fémorale après impaction, pendant les mouvements de la marche.
- meilleure tenue en fatigue de l'articulation ainsi prothésée.
- meilleure congruence de la tige à l'os.

Pour résoudre de tels problèmes il a été conçu et mis au point un ensemble prothétique de la hanche remarquable en ce qu'il comprend :
- deux familles de tiges fémorales correspondant chacune aux fémurs droits sensiblement rectilignes et aux fémurs courbes,
- les tiges fémorales de chacune des familles présentant, au niveau de leur partie proximale, d'une part, dans le plan sagittal, une courbe antérieure concave, et, d'autre part, dans le plan frontal, une courbure interne concave,
- dans le plan sagittal, le rayon de courbure antérieur est le même pour chacune des deux familles, tandis que dans le plan frontal, le rayon de courbure interne n'est pas le même pour chacune des deux familles pour correspondre à la face interne des fémurs droits et courbes
- dans le plan frontal, le rayon de courbure des tiges fémorales correspondant à la famille des fémurs courbes est inférieur à celui des tiges fémorales de la famille des fémurs droits, de sorte que la courbure interne des tiges fémorales des fémurs courbes est plus accentuée que celle des tiges fémorales des fémurs droits.

Il en résulte que la forte courbure antérieure au niveau de la partie proximale de la tige, épouse parfaitement la courbure interne du fémur, qu'il soit droit ou courbe, en supprimant, dans ces conditions, l'éventuelle absence de contact avec le merkel que seule une collerette pouvait compenser.

Toujours pour résoudre le problème posé de tenir compte de la morphologie du fémur, les tiges de chaque famille présentent un col latéralisé qui est décalé angulairement dans le plan sagittal par rapport à l'axe de symétrie , pour correspondre à l'angle d'antéversion de la tête du fémur.

Compte-tenu de la différence morphologique entre un fémur droit et un fémur courbe, dans le plan frontal, l'angle cervico-diaphysaire des tiges fémorales de la famille des fémurs à grande courbure interne est inférieur à celui des tiges fémorales de la famille des fémurs droits.

Avantageusement, l'angle cervico diaphysaire des tiges fémorales de la famille des fémurs à grande courbure interne est compris entre 125 et 130° en étant égale de préférence à 128°, tandis que l'angle cervico-diaphysaire des tiges fémorales de la famille des fémurs droits est compris entre 130 et 135° en étant égale de préférence à 134°.

Suivant une autre caractéristique, la partie distale des tiges fémorales de chacune des familles est indépendante du rayon de courbure, tant dans le plan frontal, que dans le plan sagittal.

Pour résoudre le problème posé de respecter l'anatomie du patient, dans chacune des deux familles, les tiges fémorales sont de différentes tailles et d'orientation déterminée pour correspondre au fémur côté droit et au fémur côté gauche.

L'invention est exposée ci-après plus en détail à l'aide des dessins annexés dans lesquels :
La figure 1 est une vue de face d'une tige fémorale correspondant à un fémur droit.
La figure 2 est une vue de face d'une tige fémorale de taille identique à celle illustrée figure 1, mais conformée pour être impactée dans un fémur courbe.
La figure 3 est une vue de profil de la tige fémorale correspondant à l'une quelconque des tiges fémorales représentées aux figures 1 et 2.
La figure 4 montre, par une vue de face, une tige fémorale exécutée dans une certaine taille et correspondant au fémur droit, et sur laquelle on a superposé une tige fémorale de même taille, mais correspondant à un fémur courbe, de manière à bien montrer les différences qui existententre ces deux familles de tiges ; la tige fémorale pour fémur courbe étant représentée en traits mixtes.

Selon l'invention, l'ensemble prothétique comprend deux familles de tiges fémorales pour correspondre chacune aux fémurs droits et aux fémurs courbes. Les tiges fémorales (1) de la famille des fémurs droits et les tiges fémorales (2) de la famille des fémurs courbes, présentent chacune, au niveau de leur partie proximale, d'une part, dans le plan sagittal, une courbure antérieure, et, d'autre part, dans le plan frontal, une courbure interne. La courbure antérieure dans le plan sagittal est désignée par (1a-2a), tandis que la courbure interne dans le plan frontal est désignée par (1b-2b).

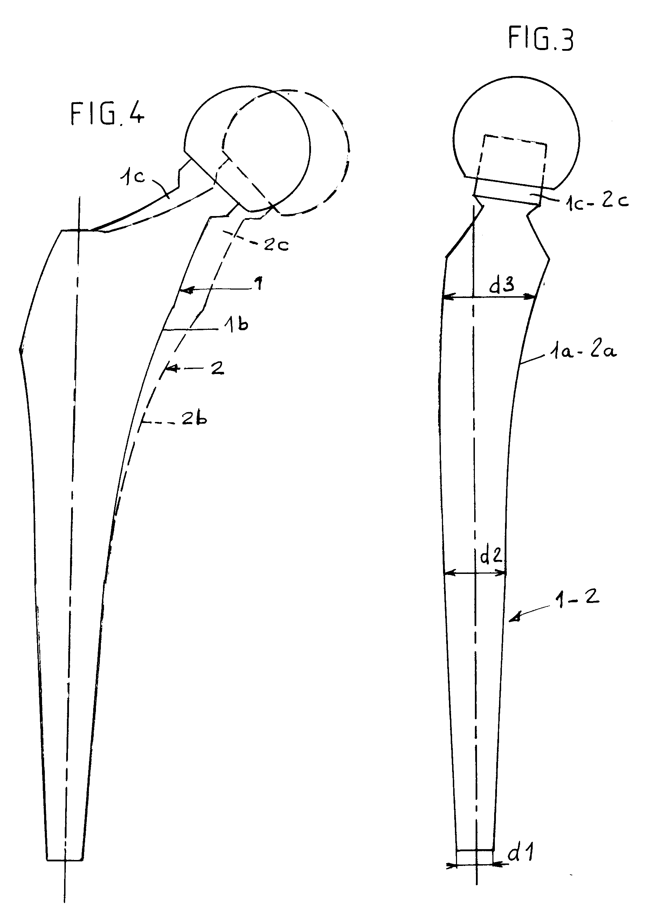

Comme le montre la figure 3, le rayon de courbure antérieur (1a-2a), dans le plan sagittal, est le même aussi bien pour une tige fémorale de la famille des fémurs droits, que pour une tige fémorale de la famille des fémurs courbes. Par contre, dans le plan frontal, le rayon de courbure interne (1b-2b) n'est pas le même pour chacune des deux familles, afin de correspondre à la face intéro-externe des fémurs droits et courbes.

Dans le plan frontal, le rayon de courbure (R) des tiges fémorales correspondant à la famille des fémurs courbes, est inférieur au rayon (R1) des tiges fémorale de la famille des fémurs droits. Dans ces conditions, comme le montre notamment la figure 4, il apparait que la courbure interne (2b) des tiges fémorales des fémurs courbes est plus accentuée que la courbure interne (1b) des tiges fémorales des fémurs droits.

Il en résulte une différence de l'angle cervico-diaphysaire. L'angle cervico-diaphysaire (A) des tiges fémorales de la famille des fémurs courbes est inférieur à l'angle cervico-diaphysaire (B) des tiges fémorales de la famille des fémurs droits. Notamment, l'angle (A) est compris entre 125 et 130°, tandis que l'angle (B) est compris entre 130 et 135°. Plus particulièrement, de manière préférée quine saurait toutefois être considérée comme rigoureusement limitative, l'angle cervico-diaphysaire (A) est égale à 128°, tandis que l'angle cervico-diaphysaire (B) est égale à 134°.

Suivant une autre caractéristique, et comme le montre la figure 3, les tiges (1) et (2) de chacune des deux familles présentent un col latéralisé (1c-2c) qui est décalé angulairement dans le plan sagittal par rapport à l'axe de symétrie, en combinaison avec la courbure interne (1a-2a). Ce décalage angulaire du col correspond à l'angle d'antéversion de la tête du fémur.

Il est à noter que la partie distale des tiges fémorales (1) et (2) de chacune des familles est indépendante des rayons de courbures internes, tant dans le plan frontal (1b-2b) que dans le plan sagittal (1a-2a).

Bien évidemment, dans chacune des familles correspondant comme indiqué, respectivement, à des fémurs droits et à des fémurs courbes, les tiges fémorales (1) et (2) sont de différentes tailles. En outre, compte-tenu de la courbure interne (1a-2a) des tiges (1) et (2), notamment dans le plan sagittal, chaque famille comprend, pour chacune des tailles, des tiges fémorales devant être impactées dans le canal médullaire des fémurs côté droit et des fémurs côté gauche.

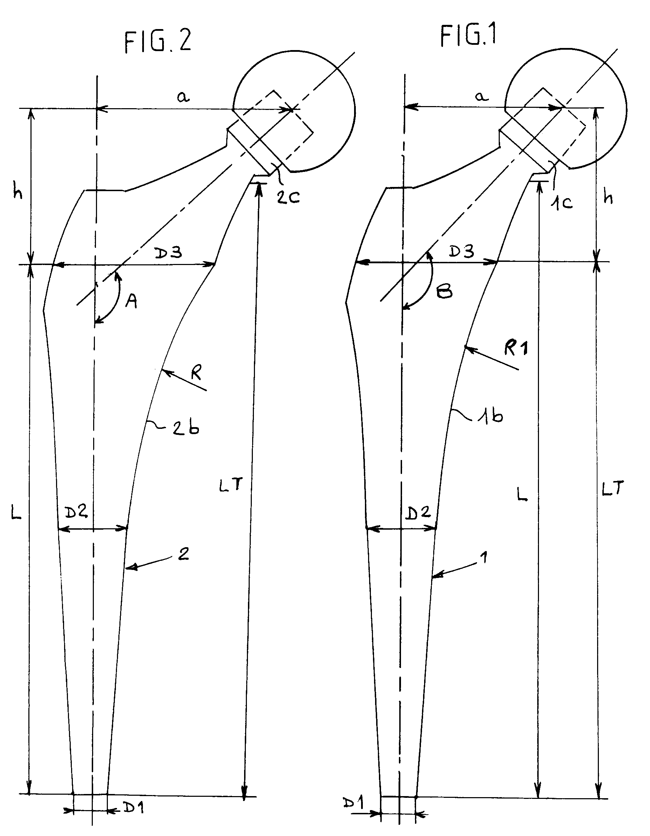

On indique, ci-après, les caractéristiques dimensionnelles des différentes tiges fémorales pour chacune des deux familles et selon différentes tailles. On se réfère, à cet égard, aux figures 1 et 2 où sont inscrits les principales références correspondant aux dimensions caractéristiques ci-après, considérées pour quatre tailles différentes, respectivement indiquées Taille 1, Taille 2, Taille 3 et Taille 4. Bien évidemment, ces caractéristiques dimensionnelles constituent des exemples-préférés d'exécution qui, cependant, ne seraient être considérés comme limitatifs.

### # TAILLE 1

| Tige fémorale pour fémurs droits : | |
|---|---|
| D1 = | 4 mm |
| D2 = | 10 mm |
| D3 = | 22,5 mm |
| h = | 41,6 mm |
| R1 = | 183 mm |
| B = | 134° |
| a = | 34,5 mm |
| H = | 33,3 mm |
| d1 = | 4 mm |
| d2 = | 7 mm |
| d3 = | 10,75 mm |
| L = | 105 mm |
| LT = | 120 mm |

| Tige fémorale pour fémurs courbes : | |
|---|---|
| D1 = | 4 mm |
| D2 = | 10 mm |
| D3 = | 26,5 mm |
| h = | 41 mm |
| R1 = | 125 mm |
| B = | 128° |
| a = | 42, 6 mm |
| H = | 34, 6 mm |
| d1 = | 4 mm |
| d2 = | 7 mm |
| d3 = | 10,75 mm |
| L = | 105 mm |
| LT = | 123 mm |

### # TAILLE 2

| Tige fémorale pour fémurs droits : | |
|---|---|
| D1 = | 5,5 mm |
| D2 = | 12,5 mm |
| D3 = | 24 mm |
| h = | 37,5 mm |
| R1 = | 183 mm |
| B = | 134° |
| a = | 37 mm |
| H = | 35,8 mm |
| d1 = | 5,5 mm |
| d2 = | 9,5 mm |
| d3 = | 14,5 mm |
| L = | 110 mm |
| LT = | 127 mm |

| Tige fémorale pour fémurs courbes : | |
|---|---|
| D1 = | 5,5 mm |
| D2 = | 12,5 mm |
| D3 = | 30 mm |
| h = | 31 mm |
| R1 = | 125 mm |
| B = | 128° |
| a = | 45,8 mm |
| H = | 35,8 mm |
| d1 = | 5,5 mm |
| d2 = | 9,5 mm |
| d3 = | 14,5 mm |
| L = | 110 mm |
| LT = | 131 mm |

### # TAILLE 3

| Tige fémorale pour fémurs droits : | |
|---|---|
| D1 = | 5,5 mm |
| D2 = | 13,5 mm |
| D3 = | 27 mm |
| h = | 41 mm |
| R1 = | 183 mm |
| B = | 134° |
| a = | 39,7 mm |
| H = | 38,3 mm |
| d1 = | 5,5 mm |
| d2 = | 12 mm |
| d3 = | 18 mm |
| L = | 120 mm |
| LT = | 139 mm |

| Tige fémorale pour fémurs courbes : | |
|---|---|
| D1 = | 5,5 mm |
| D2 = | 13,5 mm |
| D3 = | 34 mm |
| h = | 35 mm |
| R1 = | 125 mm |
| B = | 128° |
| a = | 46,9 mm |
| H = | 36,6 mm |
| d1 = | 5,5 mm |
| d2 = | 12 mm |
| d3 = | 18 mm |
| L = | 120 mm |
| LT = | 144 mm |

### # TAILLE 4

| Tige fémorale pour fémurs droits : | |
|---|---|
| D1 = | 7,5 mm |
| D2 = | 15,5 mm |
| D3 = | 31,5 mm |
| h = | 37 mm |
| R1 = | 183 mm |
| B = | 134° |
| a = | 39,7 mm |
| H = | 38,3 mm |
| d1 = | 7,5 mm |
| d2 = | 14 mm |
| d3 = | 21,5 mm |
| L = | 130 mm |
| LT = | 152 mm |

| Tige fémorale pour fémurs courbes : | |
|---|---|
| D1 = | 7,5 mm |
| D2 = | 15,5 mm |
| D3 = | 39 mm |
| h = | 34 mm |
| R1 = | 125 mm |
| B = | 128° |
| a = | 48 mm |
| H = | 37,5 mm |
| d1 = | 7,5 mm |
| d2 = | 14 mm |
| d3 = | 21,5 mm |
| L = | 130 mm |
| LT = | 158 mm |

Compte-tenu des caractéristiques sus-indiquées, et de cette classification des tiges fémorales selon deux grandes familles, il en résulte une gamme non homothétique, dont les avantages sont rappelés ci-après :
- les différents modèles ne se différencient que par la courbure interne dans le plan frontal, ce qui permet d'avoir une gamme complète où l'angle cervico-diaphysaire est variable entre les différents modèles. Il en résulte, dans une même gamme, des tiges fémorales avec des cols plus ou moins latéralisés selon les tailles.
- la forte courbure antérieure de l'implant dans le plan frontal épouse parfaitement la courbure interne du fémur, permettant ainsi de supprimer l'absence de contact osseux au niveau du merkel même sans collerette d'appui.
- l'ensemble prothétique permet de proposer, dans une gamme homogène, différentes tiges fémorales qui épousent parfaitement la partie proximale de l'os à prothéser, et par conséquent, d'améliorer la coaptation os-prothèse, en tenant compte des différences morphologiques essentielles des fémurs classés en deux grandes familles : fémurs droits - fémurs courbes.

## Revendications

1. Ensemble prothétique de la hanche, caractérisé en ce qu'il comprend :
- deux familles de tiges fémorales (1) et (2) correspondant chacune aux fémurs droits sensiblement rectilignes et aux fémurs courbes,
- les tiges fémorales (1) et (2) de chacune des familles présentant, au niveau de leur partie proximale, d'une part, dans le plan sagittal, une courbure antérieure concave (1a-2a), et, d'autre part, dans le plan frontal, une courbure interne concave (1b-2b),
- dans le plan sagittal, le rayon de courbure antérieur est le même pour chacune des deux familles, tandis que dans le plan frontal, le rayon de courbure interne n'est pas le même pour chacune des deux familles pour correspondre à la face interne des fémurs droits et courbes,
- dans le plan frontal, le rayon de courbure (R) des tiges fémorales (2) correspondant à la famille des fémurs courbes est inférieur à celui des tiges fémorales (1) de la famille des fémurs droits, de sorte que la courbure interne (2b) des tiges fémorales des fémurs courbes est plus accentuée que celle des tiges fémorales (1) des fémurs droits.

2. Ensemble selon la revendication 1, caractérisé en ce que les tiges (1) et (2) de chaque famille présentent un col latéralisé (1c-2c) qui est décalé aungulairement dans le plan sagittal par rapport à l'axe de symétrie, pour correspondre à l'angle d'antéversion de la tête du fémur.

3. Ensemble selon la revendication 1, caractérisé en ce que dans le plan frontal l'angle cervico-diaphysaire (A) des tiges fémorales de la famille des fémurs à grande courbure interne est inférieur à celui des tiges fémorales (1) de la famille des fémurs droits.

4. Ensemble selon la revendication 3, caractérisé en ce que l'angle cervico diaphysaire des tiges fémorales de la famille des fémurs à grande courbure interne est compris entre 125 et 130° en étant égale de préférence à 128°, tandis que l'angle cervico-diaphysaire des tiges fémorales de la famille des fémurs droits est compris entre 130 et 135° en étant égale de préférence à 134°.

5. Ensemble selon la revendication 1, caractérisé en ce que la partie distale des tiges fémorales (1) et (2) de chacune des familles est indépendante du rayon de courbure, tant dans le plan frontal que sagittal.

6. Ensemble selon la revendication 1, caractérisé en ce que dans chacune des deux familles, les tiges fémorales sont de différentes tailles et d'orientation déterminée pour correspondre au fémur côté droit et au fémur côté gauche.

## Claims

1. A hip prosthetic device characterised in that it consists of:
- two sets of femoral pins (1) and (2), each corresponding to straight femurs and curved femurs;
- said femoral pins (1) and (2) of each set presenting, in their proximal portion, first, in the sagittal plane, a concave anterior curvature (1a - 2a) and, secondly, in the frontal plane, a concave internal curvature (1b - 2b);
- in the sagittal plane the anterior radius of curvature is the same for each of the two sets, whilst in the frontal plane, the internal radius of curvature is not the same for each of the two sets in order to suit the internal aspects of straight and curved femurs;
- in the frontal plane the radius of curvature (R) of said femoral pins (2) corresponding to the set of curved femurs is smaller than that of said femoral pins (1) of the set of straight femurs, so that the internal curvature (2b) of femoral pins for curved femurs is greater than that of femoral pins (1) for straight femurs.

2. A prosthetic device as claimed in claim 1, characterised in that said pins (1) and (2) of each set comprise a lateral neck (1c - 2c) with angular deviation to the axis of symmetry in the sagittal plane, suiting the anteversion angle of the femoral head.

3. A prosthetic device as claimed in claim 1, characterised in that, in the frontal plane, the cervico-diaphyseal angle (A) of said sets of femoral pins for femurs with large internal curvature is smaller than that of said sets of femoral pins (1) for straight femurs.

4. A prosthetic device as claimed in claim 3, characterised in that the cervico-diaphyseal angle of femoral pins of the set for femurs with large internal curvature ranges from 125 to 130°, with a preference for a 128° angle, whilst the cervico-diaphyseal angle of femoral pins for straight femurs ranges from 130 to 135°, with a preference for a 134° angle.

5. A prosthetic device as claimed in claim 1, characterised in that the distal portion of said femoral pins (1) and (2) of each set is independent of the radius of curvature, both in the frontal and sagittal planes.

6. A prosthetic device as claimed in claim 1, characterised in that in each of the two sets, said femoral pins have different sizes and an orientation so determined as to suit the left femur or the right femur.

## Patentansprüche

1. Hüftprothesenkonstruktion bestehend aus:
- zwei Familien von Femurschäften (1) und (2), die jeweils den ungefähr geraden und den gebogenen Oberschenkelknochen entsprechen,
- wobei die Femurschäfte (1) und (2) beider Familien im Bereich ihres proximalen Teils zum einen in der sagittalen Ebene eine konkave vordere Krümmung (1a-2a) und zum anderen in der frontalen Ebene eine konkave innere Krümmung (1b-2b) aufweisen;
- in der sagittalen Ebene ist der vordere Krümmungsradius bei beiden Familien gleich, während der innere Krümmungsradius in der frontalen Ebene bei den jeweiligen Familien unterschiedlich beschaffen ist, um der Innenfläche der geraden bzw. gebogenen Oberschenkelknochen zu entsprechen;
- in der frontalen Ebene ist der Krümmungsradius (R) der der Familie der gebogenen Oberschenkelknochen entsprechenden Femurschäfte (2) geringer als derjenige der Femurschäfte (1) der Familie der geraden Oberschenkelknochen, so daß die innere Krümmung (2b) der Femurschäfte der gebogenen Oberschenkelknochen stärker ausgeprägt ist als bei den Femurschäften (1) der geraden Oberschenkelknochen.

2. Prothesenkonstruktion nach Anspruch 1, dadurch gekennzeichnet, daß die Schäfte (1) und (2) der jeweiligen Familie einen seitlichen Hals (1c-2c) aufweisen, der in der sagittalen Ebene gegenüber der Symmetrieachse im Winkel versetzt ist, um dem Neigungswinkel des Femurkopfes zu entsprechen.

3. Prothesenkonstruktion nach Anspruch 1, dadurch gekennzeichnet, daß der zerviko-diaphysäre Winkel (A) der Femurschäfte der Familie der Oberschenkelknochen mit großer Innenkrümmung in der frontalen Ebene kleiner ist als derjenige der Femurschäfte (1) der Familie der geraden Oberschenkelknochen.

4. Prothesenkonstruktion nach Anspruch 3, dadurch gekennzeichnet, daß der zerviko-diaphysäre Winkel der Femurschäfte der Familie der Oberschenkelknochen mit großer Innenkrümmung 125-130° (vorzugsweise 128°) beträgt, während der zerviko-diaphysäre Winkel der Femurschäfte der Familie der geraden Oberschenkelknochen 130-135° (vorzugsweise 134°) beträgt.

5. Prothesenkonstruktion nach Anspruch 1, dadurch gekennzeichnet, daß der distale Teil der Femurschäfte (1) und (2) beider Familien sowohl in der frontalen wie in der sagittalen Ebene vom Krümmungsradius unabhängig ist.

6. Prothesenkonstruktion nach Anspruch 1, dadurch gekennzeichnet, daß die Femurschäfte in beiden Familien unterschiedlich groß und so ausgerichtet sind, um dem Oberschenkelknochen auf der rechten Seite und dem Oberschenkelknochen auf der linken Seite zu entsprechen.
